Europäisches Patentamt

**European Patent Office**

Office européen des brevets

(19)

(11) Numéro de publication: **0 024 253**
**A1**

(12) # DEMANDE DE BREVET EUROPEEN

(21) Numéro de dépôt: **80810188.5**

(22) Date de dépôt: **03.06.80**

(51) Int. Cl.³: **A 61 F 5/44**

(30) Priorité: **08.08.79 CH 7278/79**

(43) Date de publication de la demande:
**25.02.81 Bulletin 81/8**

(84) Etats Contractants Désignés:
**BE DE FR GB IT NL SE**

(71) Demandeur: **De Zaepffel, Brigitte**
**c/o M.B.S. Bd James-Fazy 6**
**CH-1201 Genève(CH)**

(72) Inventeur: **De Zaepffel, Brigitte**
**c/o M.B.S. Bd James-Fazy 6**
**CH-1201 Genève(CH)**

(54) **Poche iléostomique.**

(57) La poche comprend un sac souple (1) fixé à une ouverture centrale d'une feuille de fixation souple (2). La feuille de fixation est munie d'une couche auto-adhésive entourant immédiatement l'ouverture et revêtue d'une pellicule anti-adhésive amovible (9) et d'une couche auto-adhésive, également revêtue d'une pellicule anti-adhésive amovible (10), concentrique à la première et suivant le pourtour de la feuille de fixation.

On peut retirer à volonté soit la pellicule (9), soit la pellicule (10) et utiliser ainsi tour à tour des surfaces distinctes de peau pour coller la poche, ce qui combat l'irritation de la peau.

**FIG.2**

EP 0 024 253 A1

-1-

## Poche iléostomique

La présente invention a pour objet une poche ostomique (iléostomique ou colostomique, voire caecostomique) plastique auto-adhésive.

On utilise couramment des poches plastiques auto-adhésives pour recueillir les matières fécales s'écoulant des anus artificiels. L'utilisateur, après avoir retiré la pellicule anti-adhésive qui revêt la couche auto-adhésive, applique celle-ci sur la peau entourant l'anus artificiel. La couche auto-adhésive assure l'étanchéité du joint entre la peau et la poche. Lorsque la poche est pleine ou lorsque le patient veut en changer pour une raison quelconque, l'utilisateur doit l'arracher de la peau et en placer une nouvelle. Comme c'est toujours la même surface de peau qui reçoit la couche adhésive, cette surface de peau s'irrite progressivement et finalement l'emploi de poches auto-adhésives devient pénible ou même impossible.

La poche qui fait l'objet de l'invention vise à pallier cet inconvénient. Suivant l'invention, la feuille de fixation de la poche sur la peau présente au moins deux zones auto-adhésives distinctes, s'étendant sans interruption autour de l'orifice d'entrée des matières; ces zones concentriques sont séparées par une zone non adhésive, et elles sont revêtues d'une pellicule anti-adhésive amovible.

L'objet de la présente invention est donc une poche ostomique, plastique, auto-adhésive, caractérisée par le fait qu'elle comporte un sac souple (1) en matière plastique, transparent ou opaque et fixé à l'ouverture d'une feuille de fixation (2) souple, munie de deux plages auto-adhésives, concentriques, dont l'une entoure immédiatement l'ouverture à laquelle est fixé le sac souple et dont l'autre est séparée de la première par une zone intermédiaire, non adhésive, en matériel absorbant qui peut contenir un produit bactéricide ou cicatrisant, les deux couches auto-adhésives étant revêtues chacune d'une pellicule (9,10) anti-adhérente et amovible.

Le dessin annexé représente, à titre d'exemple, une forme d'exécution de la poche, objet de l'invention.

Les fig. 1 et 2 sont des vues, respectivement en perspective et en plan, de la poche encore munie des pellicules anti-adhésives amovibles.

Les fig. 3 et 4 sont des vues, respectivement en plan et en coupe axiale, de la poche après élimination des pellicules anti-adhésives.

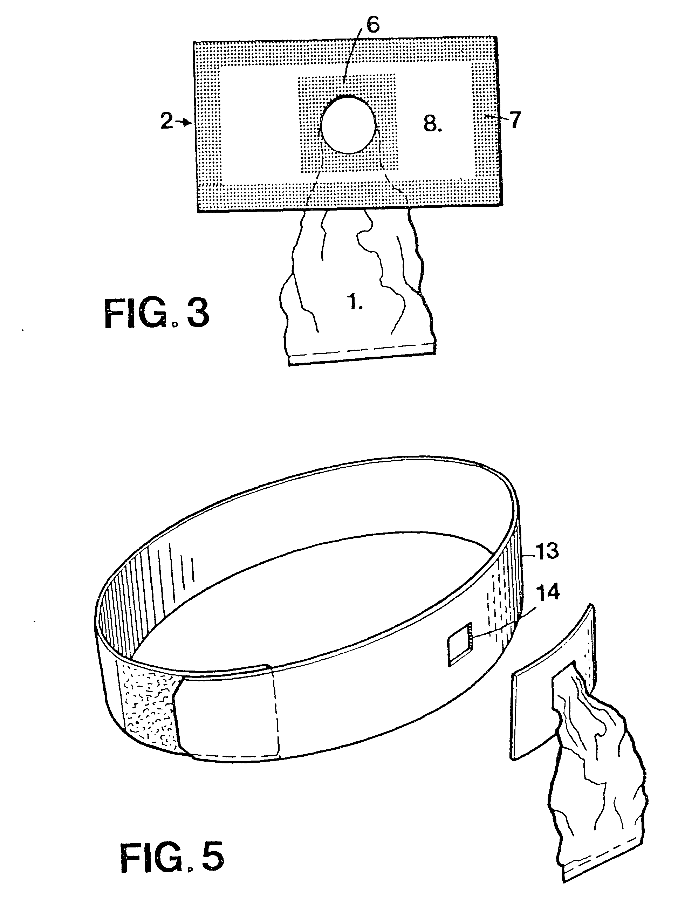

La fig. 5 montre une ceinture servant à maintenir la poche contre l'abdomen.

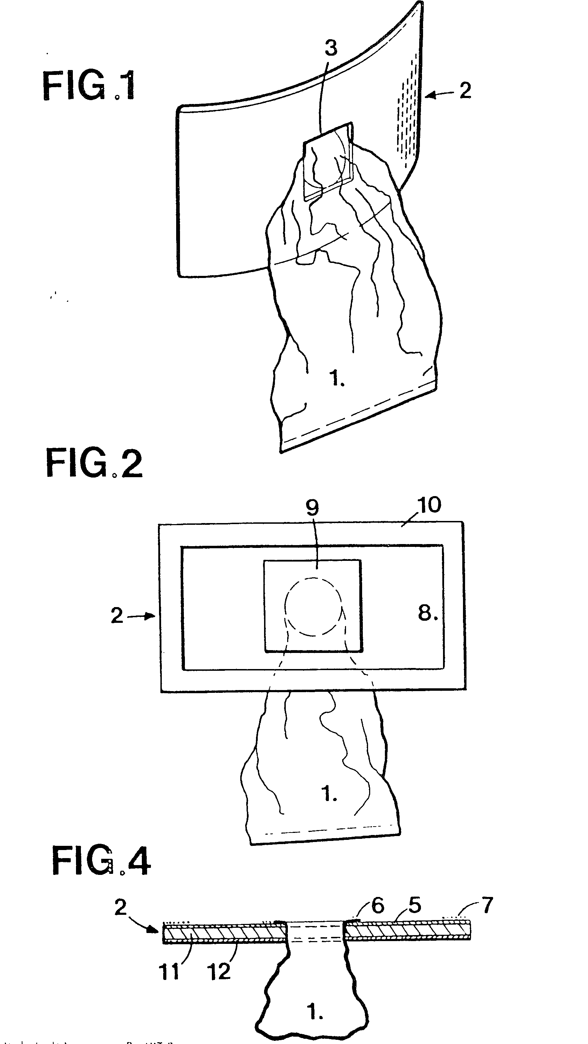

La poche représentée comprend un sac 1 en pellicule de matière plastique dont la base est fermée par une ligne de soudure et dont l'entrée passe à travers l'orifice central 3 d'une feuille souple de fixation 2. Le bord de l'entrée du sac est rabattu au bord de l'orifice 3 et est collé sur la couche interne 5 de la feuille 2. Sur sa face destinée à être appliquée sur la peau, la feuille 2 est munie de deux zones distinctes de couche auto-adhésive 6 et 7 (fig.3 et 4) concentriques s'étendant sans interruption autour de l'orifice 3 d'entrée des matières et séparées par une zone non adhésive 8. Sur la fig. 2, on voit ces couches auto-adhésives encore revêtues de pellicules anti-adhésives amovibles 9 et 10.

La feuille de fixation 2 est représentée à la fig. 4 avec une épaisseur exagérée pour la clarté du dessin. Cette feuille est constituée de plusieurs couches superposées comprenant, de haut en bas sur la fig. 4, un voile poreux 5 de fibres par exemple d'ouate, dont les fibres du moins en surface ont été recouvertes de vapeurs d'aluminium, une couche de cellulose absorbante 11 et une pellicule imperméable 12. L'étanchéité du pourtour de la couche 11 est assurée par des moyens non représentés, tels qu'une pellicule imperméable repliée en forme de U et appliquée sur le pourtour de la feuille 2 en dessous de la couche auto-adhésive 7.

Le sac plastique 1 et la feuille imperméable peuvent être faits d'une matière polymère, convenable quelconque qui est à l'épreuve de l'humidité et des odeurs et qui possède la résistance mécanique nécessaire. Des matières convenables comprennent par exemple le polyéthylène, les copolymères du chlorure et de l'acétate de vinyle avec le chlorure de polyvinylidène etc ainsi que les stratifiés de ces matières, par exemple les stratifiés de polyvinylacétate ou de polyéthylène et de copolymères du chlorure de vinyle et de chlorure de polyvinylidène. Sac et pellicule peuvent être transparents ou opaques. Leur épaisseur varie en fonction de la matière polymère particulière, mais sera généralement comprise entre 0,05 et 0,3 mm environ.

Les plages ou zones auto-adhésives peuvent être de forme rectangulaire comme indiqué aux figures 2 et 3. Elles peuvent aussi bien prendre une forme circulaire ou élipsoïdique voire toute autre forme appropriée. Ces plages sont en général constituées d'une simple bande auto-collante dont une des faces adhère à la feuille de fixation, l'autre étant recouverte d'une pellicule (9 ou 10) non adhésive, par exemple d'une mince feuille de polyéthylène ou de papier siliconé qui protège la partie adhérente de la zone collante. Il va de soi qu'on choisira de préférence une colle qui n'irrite pas la peau.

La zone absorbante située entre les deux plages adhérentes

-4-

de la poche selon la présente invention est constituée d'un matériel fibreux (gaze, tulle ou ouate) qui peut contenir une
poudre médicinale ou un désinfectant, voire un produit qui favorise la cicatrisation ou qui calme la peau éventuellement irritée. D'excellents résultats sont obtenus avec les produits du
type des "métallines" de la maison Lohmann KG, qui sont des tissus, des feutres ou des non tissés dont les fibres ont été recouvertes de vapeur d'aluminium.

Pour l'utilisation de la poche, on peut retirer soit la pellicule 9 soit la pellicule 10 puis appliquer la feuille 2 sur la
peau de manière que l'orifice 3 soit en regard de l'anus artificiel et que la zone auto-adhésive dont on a retiré la pellicule
anti-adhérente puisse se coller à la peau. Lorsque c'est la pellicule 9 qui a été retirée c'est la zone intérieure 6 qui est
active et le dispositif fonctionne comme une poche de type connu.
Lorsque c'est la pellicule 10 qui a été retirée c'est la zone
auto-collante extérieure 7 qui est active et la surface de peau
qui entoure immédiatement l'anus artificiel peut se reposer.
Dans cette situation, les matières fluides peuvent s'insinuer
entre la feuille de fixation 2 et la peau jusqu'au bord interne
de la zone 7. Toutefois, grâce à l'action bactéricide douce du
voile de fibre d'aluminium 5 et du pouvoir absorbant de la couche de cellulose 11, la surface de peau comprise entre l'anus
artificiel et la zone 7 n'est pas affectée.

Pour assurer le maintien de la poche sur le corps de l'utilisateur, on emploie une ceinture 13 de type connu, à fermeture
à poils rigides dite "Velcro" ou toute autre ceinture adéquate.
On passe le sac 1 de l'intérieur à l'extérieur à travers un trou
14 pratiqué à l'emplacement approprié dans la ceinture 13, trou
dont le pourtour correspond à celui de l'orifice 3, la feuille
de fixation restant serrée sous la ceinture contre le corps du
patient lorsque celui-ci a bouclé sa ceinture.

# Revendications

1.- Poche ostomique, plastique, auto-adhésive, caractérisée par le fait qu'elle comporte un sac souple (1) en matière plastique, transparent ou opaque et fixé à l'ouverture d'une feuille de fixation (2) munie de deux plages auto-adhésives, concentriques, dont l'une entoure immédiatement l'ouverture à laquelle est fixé le sac souple et dont l'autre est séparé de la première par une zone intermédiaire, non adhérente, en matériel absorbant qui peut contenir un produit bactéricide ou cicatrisant, les deux zones auto-adhésives étant revêtues chacune d'une pellicule anti-adhésive amovible (9, 10).

2.- Poche iléostomique selon la revendication 1 caractérisée par le fait que la zone intermédiaire située entre les deux plages auto-adhésives est en fibres cellulosiques.

3.- Poche iléostomique selon la revendication 1 caractérisée par le fait que la zone intermédiaire située entre les deux plages auto-adhésives est une gaze ou un tulle comportant une poudre médicinale, un désinfectant ou un antibiotique.

4.- Poche iléostomique selon la revendication 1 caractérisée par le fait que la zone intermédiaire située entre les deux plages auto-adhésives est faite d'ouate ou d'un tissu absorbant, en matière fibreuse dont les fibres sont recouvertes d'aluminium.

5.- Poche iléostomique selon la revendication 1, caractérisée par le fait que la plage auto-adhésive qui entoure immédiatement l'orifice de la feuille de fixation sur la peau est une plage ininterrompue.

6.- Combinaison d'une poche ostomique et d'une ceinture de maintien, caractérisée par le fait que la ceinture est percée d'un trou (14) placé et dimensionné de manière que la poche puisse être disposée à travers ledit trou en ayant son orifice d'entrée en regard de l'anus artificiel du porteur et que la feuille de fixation sur la peau soit serrée contre la peau du porteur lorsque celui-ci a fermé la ceinture.

**FIG.1**

**FIG.2**

**FIG.4**

FIG. 3

FIG. 5

**Office européen des brevets**

**RAPPORT DE RECHERCHE EUROPEENNE**

Numéro de la demande
EP 80 81 0188

## DOCUMENTS CONSIDERES COMME PERTINENTS

| Catégorie | Citation du document avec indication, en cas de besoin, des parties pertinentes | Revendica-tion concernée |
|---|---|---|
| X | US - A - 3 081 771 (LEE) <br> * Colonne 1, lignes 16-24, 61-70; colonne 2, lignes 44-62; figures 1,4 * | 1,5 |
| | -- | |
| | GB - A - 1 328 764 (MARSAN) <br> * Page 4, colonne 2, lignes 92-110, 122-127; page 5, colonne 1, lignes 48-50; figures 8,17 * | 1,5 |
| | -- | |
| | DE - A - 2 722 445 (HILGERS) <br> * Page 6, ligne 21 - page 7, ligne 12; figures 1-4 * | 1,6 |
| | -- | |
| | US - A - 2 896 625 (AUSTIN) <br> * Colonne 2, lignes 16-41; figure 3 * | 3 |
| | -- | |
| | FR - A - 2 246 282 (HOLLISTER) <br> * Page 2, lignes 24-37; page 3, lignes 1-5; figures 1,2 * | 1 |
| | ---- | |

**CLASSEMENT DE LA DEMANDE (Int. Cl. ³)**

A 61 F 5/44

**DOMAINES TECHNIQUES RECHERCHES (Int. Cl. ³)**

A 61 F

**CATEGORIE DES DOCUMENTS CITES**

X particulièrement pertinent
A. arriere-plan technologique
O: divulgation non-ecrite
P document intercalaire
T. théorie ou principe a la base de l invention
E. demande faisant interference
D: document cité dans la demande
L document cite pour d'autres raisons

&: membre de la même famille, document correspondant

X | Le présent rapport de recherche a été établi pour toutes les revendications

| Lieu de la recherche <br> La Haye | Date d'achèvement de la recherche <br> 28-10-1980 | Examinateur <br> PASTUREL |
|---|---|---|

OEB Form 1503.1 06.78